# EUROPEAN PATENT APPLICATION

(11) **EP 4 595 879 A1**
(43) Date of publication of application: **06.08.2025**
(21) Application number: 22960620.7
(22) Date of filing: 02.12.2022
(51) Int. Cl.: A61B 5/145, A61F 13/02

(54) **REINFORCEMENT PATCH**

(30) Priority: 29.09.2022 CN 202222601229 U
(71) Applicant: Shenzhen Sisensing Co., Ltd., Shenzhen, Guangdong 518110 (CN)
(72) Inventor: LI, Yunfeng, Shenzhen,Guangdong 518110 (CN); WU, Jiang, Shenzhen,Guangdong 518110 (CN); CHEN, Sai, Shenzhen,Guangdong 518110 (CN)
(74) Representative: Isern Patentes y Marcas S.L.
(86) International application number: PCT/CN2022/136106
(87) International publication number: WO 2024/066025

(57) **Abstract**

The present disclosure describes a reinforced applicator (1), including an adhesion portion (11), a supporting portion (12), and an adhesive layer (13). The adhesion portion (11) has an upper surface and a lower surface which are opposite to each other. The Young's modulus of the supporting portion (12) is greater than the Young's modulus of the adhesion portion (11) and supports the adhesion portion (11). The supporting portion (12) has a first supporting member (121) provided on the adhesion portion (11), and a second supporting member (124) formed by extending from the first supporting member (121) along the direction away from the upper surface, the adhesive layer (13) is provided on the lower surface. According to the present disclosure, it is able to provide a reinforced applicator (1) which can be conveniently picked up and accurately reinforce an applicator in the case of one-handed operation.

## Description

### BACKGROUND OF INVENTION

### Field of Invention

The present disclosure relates to a reinforced applicator.

### Description of Related Art

For the needs of clinical diagnosis or personal health monitoring, a medical instrument is needed to be used to monitor the physiological parameters of a human body in real time. For example, for diabetics, it is quite necessary to use a blood glucose monitor to continuously monitor the glucose concentration of interstitial fluid in real time on weekdays and adjust the glucose concentration by means of adjusting diet or applying medication etc., so as to reduce and lower the occurrence of complications due to abnormal glucose concentration.

At present, some patients may use application-type blood glucose monitors for monitoring their glucose concentrations. When used, the user needs a reinforced applicator to secure the applicator of the application-type blood glucose monitor to the surface of skin. When used, a circular reinforced applicator is provided on the periphery of the applicator in sleeving manner and covers a portion of the applicator so as to secure the applicator to the surface of skin.

However, in the above-mentioned prior art, due to the softer texture of the reinforced applicator made of non-woven fabrics, it is inconvenient to operate when the reinforced applicator is picked up, meanwhile, the reinforced applicator is easy to be deformed when it is picked up, thus, it is not facilitated for the reinforced applicator to be adhered on the surface of skin flatly; moreover, when the reinforced applicator is placed on the periphery of the applicator in sleeving manner by the user with one hand, it can be hardly localized accurately and deflection is easy to happen, which may impact the secured effect of the reinforced applicator; and wrinkles are easily produced when the reinforced applicator is stuck on the surface of skin, thereby the adhesive effect is affected. In this case, a reinforced applicator which can be conveniently picked up and accurately reinforce an applicator in the case of one-handed operation is needed.

### SUMMARY OF INVENTION

The present invention is made in view of the above-mentioned situation in the prior art, and its object is to provide a reinforced applicator which can be conveniently picked up and accurately reinforce an applicator in the case of one-handed operation.

For the above purposes, the present disclosure provides a reinforced applicator, which includes an adhesion portion, a supporting portion, and an adhesive layer, the adhesion portion has an upper surface and a lower surface which are opposite to each other, a Young's modulus of the supporting portion is greater than a Young's modulus of the adhesion portion and supports the adhesion portion, the supporting portion has a first supporting member provided on the adhesion portion, and a second supporting member formed by extending from the first supporting member along the direction away from the upper surface, the adhesive layer is provided on the lower surface.

In the reinforced applicator according to the present embodiment, the Young's modulus of the supporting portion is set to be greater than the Young's modulus of the adhesion portion and support the adhesion portion, thereby enabling the deformation resistance of the supporting portion to be greater than the deformation resistance of the adhesion portion, and it is facilitated to accurately provide the reinforced applicator on the periphery of the applicator in sleeving manner when the reinforced applicator is used. Meanwhile, the supporting portion has a first supporting member provided on the adhesion portion, and a second supporting member formed by extending from the first supporting member along the direction away from the upper surface of the adhesion portion, when the reinforced applicator is picked up, the reinforced applicator can be picked up by picking up the second supporting member which protrudes from the surface of the adhesion portion, thereby the convenience of operations can be improved, and the undesired deformation caused by picking up the reinforced applicator is reduced, thereby, it is facilitated to stick the reinforced applicator on the skin surface flatly. In addition, a certain degree of deformation resistance can be provided to the reinforced applicator by using the supporting portion, thereby it is able to flatly stick the reinforced applicator on the skin surface for long time.

In addition, in the reinforced applicator of the present embodiment, optionally, the adhesion portion is of embracing structure. In this case, the applicator is stuck on the surface of skin of a user in an embracing manner via the adhesion portion, the adhesion stability of the applicator can be improved.

In addition, in the reinforced applicator of the present embodiment, optionally, the first supporting member is provided along the inner periphery of the adhesion portion. In this case, the first supporting member with a relatively large Young's modulus is provided in the inner periphery of the adhesion portion, enabling the deformation resistance of the inner periphery of the reinforced applicator to be greater than the deformation resistance of the adhesion portion, it is facilitated for the reinforced applicator to be accurately provided on the periphery of the applicator in sleeving manner when the reinforced applicator is used.

In addition, in the reinforced applicator of the present embodiment, optionally, the first supporting member and/or the second supporting member are/is of annular shape. Thereby, the deformation resistance of inner periphery of the reinforced applicator can be further improved, and it is more convenient to align the location when the reinforced applicator is used.

In addition, in the reinforced applicator of the present embodiment, optionally, the inner diameter of the second supporting member is gradually tapered off as it is away from the upper surface. In this case, an accommodating space capable of securing and holding the reinforced applicator can be provided for the applicator via the second supporting member, the stability of the applicator can be improved when sticked, thereby effectively preventing the displacement and dropping of applicator during use.

In addition, in the reinforced applicator of the present embodiment, optionally, one end of the second supporting member away from the upper surface is of arc-shaped. In this case, one end of the second supporting member protruding from the upper surface is set to be arc-shaped, enabling the outer contour of the second supporting member to be smoother, and in the using process of a user, it is able to reduce the scratches on the reinforced applicator caused by articles, such as clothing etc., thereby it is facilitated to improve the adhesion stability of the reinforced applicator.

In addition, in the reinforced applicator of the present embodiment, optionally, the second supporting member includes a hollowout formed on the side wall. In this case, the inner space of the reinforced applicator can be connected to the outside through the hollow out, thereby it is facilitated to evaporate and emit the liquids such as sweat generated by a user's skin surface at the applicator site and occasional entering of moistures, thereby, it is facilitated to keep the position of the applicator in dry and comfortable conditions so as to improve the comfort of the user.

In addition, in the reinforced applicator of the present embodiment, optionally, the first supporting member includes an upper layer sheet and a lower layer sheet which are of sheet shape, the adhesion portion is clamped by the upper layer sheet and the lower layer sheet. In this case, the first supporting member can be connected more securely to the adhesion portion by the upper layer sheet and the lower layer sheet clamping the adhesion portion .

In addition, in the reinforced applicator of the present embodiment, optionally, a waterproof layer is provided on the upper surface. In this case, the possibility of bacterial infection at the application site caused by liquid entering the application area of the applicator can be effectively reduced.

In addition, the reinforced applicator of the present embodiment, optionally, includes a peeling layer provided on the adhesive layer, the peeling layer is provided on the surface of the adhesive layer and covers at least a portion of the adhesive layer. In this case, the adhesive layer is protected by the peeling layer, enabling to effectively prevent a decrease in adhesion strength of the adhesive layer caused by contamination prior to use, thereby it is facilitated to maintain the adhesion strength of the adhesive layer so as to improve the adhesion stability of the reinforced applicator on the skin surface of the user when used.

According to the present disclosure, it is able to provide a reinforced applicator which can be conveniently picked up and accurately reinforce an applicator in the case of one-handed operation.

### BRIEF DESCRIPTION OF THE DRAWINGS

Fig.1 is a schematic diagram showing an application scenario of the reinforced applicator according to an example of the present disclosure.
Fig.2 is a schematic diagram showing the applicator according to an example of the present disclosure.
Fig.3 is a schematic diagram showing the reinforced applicator according to an example of the present disclosure.
Fig.4 is a schematic diagram showing the supporting portion according to an example of the present disclosure.
Fig.5 is a schematic structural diagram showing the reinforced applicator according to an example of the present disclosure.
Fig.6 is a schematic explosive diagram showing the reinforced applicator according to an example of the present disclosure.

### DETAILED DESCRIPTION

Hereinafter, preferred embodiments of the present disclosure will be described in detail with reference to the accompanying drawings. In the following descriptions, the same reference numerals are given to the same parts, and repeated descriptions are omitted. In addition, the drawings are merely schematic, and the proportions of dimensions of the parts relative to one another or the shape of the parts etc. may differ from practical conditions.

It should be noted that the terms "include" and "have", and any variations thereof in the present disclosure, such as a process, method, system, product, or device that includes or has a list of steps or elements are not necessarily limited to those explicitly recited steps or elements, but may include or have other steps or elements not explicitly recited or inherent to such process, method, product, or device.

It should be noted that the terms of relative positions and relative directions thereof in this article, such as "upside", "toward upside", "downside", "toward downside", "up and down direction", "left side", "toward left side", "left", "toward left", "right side", "toward right side", "right", "toward right", "left and right direction", "ahead", "toward ahead", "rear", "toward rear" etc. are reference to the usually operational postures and should not be considered restrictive.

The present disclosure relates to a reinforced applicator, which may be used to reinforce the applicator applicated on the body surface, so that the applicator can be applicated on the body surface more securely. The reinforced applicator according to the present disclosure may also be referred to as a secured applicator, a strengthening applicator, or a reinforced applicator for the applicator etc.

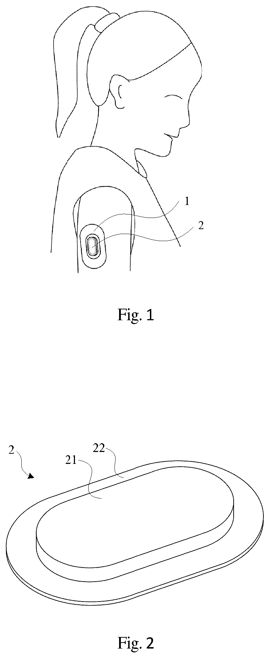

Fig.1 is a schematic diagram showing an application scenario of the reinforced applicator 1 according to an example of the present disclosure.

In the present embodiment, when the applicator 2 is placed on the arm of a user (hereinafter, also referred to "host"), the applicator 2 may be further reinforced by a reinforced applicator 1 to enhance the application stability of the applicator 2 on the body surface of a host. The reinforced applicator 1 is provided on the periphery of the applicator 2 in sleeving manner and is stuck on the user's arm so as to reinforce the applicator 2 stuck on the user's arm (see Figure 1). In this case, the applicator 2 can be reinforced and stuck on the body surface of the host via the reinforced applicator 1.

Fig.2 is a schematic diagram showing the applicator 2 according to an example of the present disclosure.

In some examples, the applicator 2 may be a sensing device. In some examples, the applicator 2 may be a glucose sensor device. In this case, the sensing device is able to react with specific analytes in the body fluids and generates analyte information, thereby it is facilitated to acquire the analyte information in the body fluids. It should be noted a skilled person in the art may understand that the applicator 2 according to the present disclosure may be other devices applicated on the body surface. For example, the applicator 2 may be application devices such as a traditional Chinese medicine application, radiotherapy locating application, and transfusion needle application etc., and the applicator 2 according to the present disclosure is not limited to the examples listed above.

In some examples, the applicator 2 may include a sensor. In some examples, the sensor may be partially or completely placed subcutaneously on a host. In some examples, the sensor may be a glucose sensor. In this case, the sensor is placed subcutaneously, the sensor is capable of reacting with glucoses in the subcutaneous interstitial fluid and generating relative information of glucoses, thereby the obtainment of glucose information in the host subcutaneous interstitial fluid can be facilitated.

In some examples, the applicator 2 may include an application housing 21 and a flange 22 (see Fig.2). In some examples, the flange 22 may extend outward from the edge of the application housing 21. In some examples, the applicator 2 may be reinforced via the reinforced applicator 1 by covering the surface of the flange 22 and sticking the flange 22 on the body surface of a host. Thereby, the reinforcement of the applicator 2 can be facilitated.

In some examples, the surface of the flange 22 contacting the host and the surface of the application housing contacting the host may be on the same plane. In this case, the surface of the flange 22 contacting the host and the surface of the application housing contacting the host are provided on the same plane, resulting in improving the adhesion stability of the applicator 2 being applicated to the face of the host. However, the examples of the present disclosure are not limited hereof, in other examples, the surface of the flange 22 contacting the host and the surface of the application housing contacting the host may also not be on the same plane.

In some examples, the applicator 2 may further include an adhesion sheet having adhesiveness provided on the application housing 21. In this case, the applicator 2 can be stuck and secured on the body surface of a host via the adhesion sheet.

In some examples, the adhesion surface of the adhesion sheet may be slightly larger than the bottom surface of the application housing 21 close to the host. In some examples, a portion of the adhesive sheet extending outward from the edge of the application housing 21 may be taken as the flange 22. In other examples, the adhesion surface of the adhesive sheet may also be substantially equal to the bottom surface of the application housing 21 close to the host. Thereby, the stability of the applicator 2 can be improved when stuck to the body surface of the host.

In some examples, when the applicator 2 is used by a user, the applicator 2 may be continuously worn for a duration of one day to thirty days. In this case, the user may perform daily activities such as exercise, bathing etc. as wearing the applicator 2, which may result in unexpectedly shifting or sliding of the applicator 2, therefore, the applicator 2 needs to be further reinforced.

As mentioned above, the applicator 2 according to the present embodiment may be reinforced on the body surface of the host via reinforced applicator 1. Hereinafter, with reference to the accompanying drawings, the reinforced applicator 1 according to the present embodiment will be described in detail.

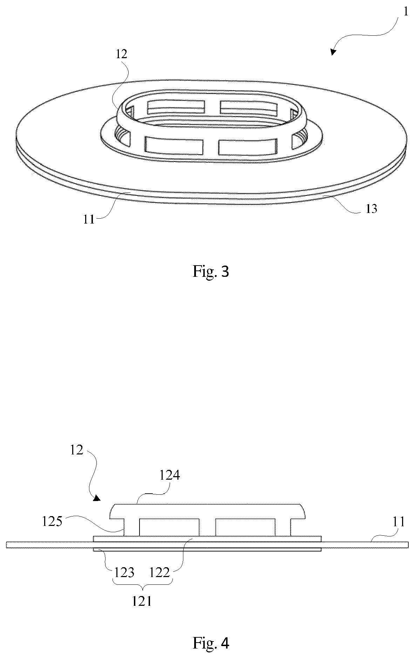

Fig.3 is a schematic diagram showing the reinforced applicator 1 according to the present disclosure.

In the present embodiment, the reinforced applicator 1 may include an adhesion portion 11 and an adhesive layer 13 (see Fig.3). In some examples, the adhesion portion 11 may have a upper surface and a lower surface which are opposite to each other. In some examples, the adhesive layer 13 may be provided on the adhesion portion 11 (see Fig.3). In some examples, the adhesive layer 13 may have adhesiveness. In this case, the adhesive layer 13 can be stuck on the skin surface of a user, and the adhesion portion 11 can also be stuck on the skin surface of the user.

In some examples, the adhesion portion 11 may be of embracing structure (see Fig.3). In this case, the applicator 11 is stuck on the skin surface of the user in an embracement manner, thereby the adhesion stability of the applicator 2 can be improved.

In some examples, the inner contour of the adhesion portion 11 may match with the outer contour of the application housing 21. In some examples, the inner contour of the adhesion portion 11 matches with the outer contour of the application housing 21 may means that the inner contour of the adhesion portion11 is substantially the same as the outer contour of the application housing 21, and the inner contour of the adhesion portion 11 is slightly larger than the outer contour of the application housing 21. Thereby, the adhesion portion 11 provided on the periphery of the application housing 21 can be facilitated, and it is facilitated to reinforce the applicator 2 by using the reinforced applicator 1.

In some examples, the inner diameter of the adhesion portion 11 may be smaller than the outer diameter of the flange 22. In this case, when the applicator 11 is provided on the periphery of the application housing 21 in sleeving manner, the applicator 2 can be further reinforced on the body surface of a host by covering a portion of the flange 22.

In some examples, the adhesion portion 11 may be a flexible object. In some examples, the adhesion portion 11 may be made of materials with flexibility. For example, the adhesion portion 11 may be made of non-woven fabrics, plain cloths etc. In some examples, the adhesion portion 11 may be of sheet shape. Thereby, the adhesion portion 11 can be applied to different adhesive locations of different users, and the comfort of adhesion can be improved.

In some examples, a waterproof layer may be provided on the upper surface of the adhesion portion 11. Specifically, the waterproof layer may be provided on the opposite side of the adhesion portion 11 applicated on the surface of a human body. In this case, the waterproof layer is provided on the upper surface of the adhesion portion 11, the adhesion portion 11 can be effectively prevented from being soaked with liquid, thus, the possibility of bacterial infection at the application site caused by liquid entering the application area of the applicator 2 can be effectively reduced.

In some examples, the adhesive layer 13 may be provided on the lower surface of the adhesion portion11. In some examples, the adhesive layer 13 has an adhesive property. In this case, the reinforced applicatorl can be stuck on the skin surface of the user via the adhesive layer 13.

In some examples, the lower surface of the adhesion portion 11 may be completely covered by the adhesive layer 13. In this case, the reinforced applicator 1 can be stuck on the skin surface more securely. However, the examples of the present disclosure are not limited hereof, in other examples, the lower surface of the adhesion portion 11 may be partially covered by the adhesive layer 13.

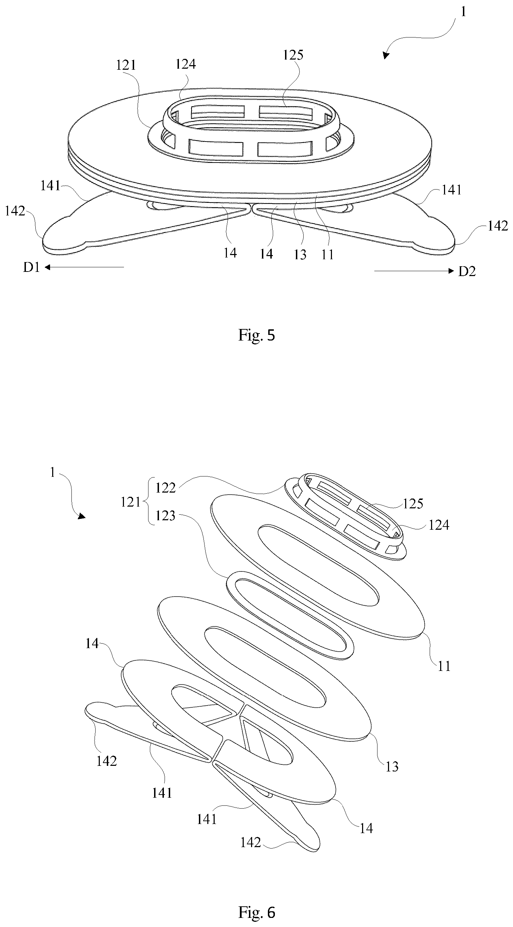

Fig.4 is a schematic diagram showing the supporting portion 12 according to an example of the present disclosure. Fig.5 is a schematic structure diagram showing the reinforced applicator 1 according to an example of the present disclosure. Wherein, the illustrative angle of view in Fig. 4 is the frontal viewing angle from the front to the back.

In some examples, the reinforced applicator 1 may further include a supporting portion 12 (see Fig.3, Fig.4).

In some examples, the supporting portion 12 may be provided on the adhesion portion 11 (see Fig.3, Fig.4). In some examples, the young's modulus of the supporting portion 12 may be greater than the young's modulus of the adhesion portion 11. In some examples, the adhesion portion 11 may be supported by the supporting portion 12. In this case, the deformation resistance of the supporting can be enabled to be greater than the deformation resistance of the adhesion portion 11, when the reinforced applicator 1 is used, due to not easy deformation of the supporting portion 12, thereby it is facilitated for the reinforced applicator 1 to be provided on the periphery of the applicator 2 in sleeving manner.

In some examples, the supporting portion 12 may have a first supporting member 121 provided on the adhesion portion 11, and a second supporting portion 124 formed by extending from the first supporting member 121 along the direction away from the upper surface (see Fig.4, Fig.5). That is, the second supporting member 124 may be provided on the adhesion portion 11 in a manner of protruding from the surface of the adhesion portion 11. In this case, when the reinforced applicator 1 is picked up, the reinforced applicator 1 can be picked by picking up the second supporting member 124 protruding from the surface of the adhesion portion 11, the convenience of operations can be improved, meanwhile, the undesired deformation of the reinforced applicator 1 can be reduced when it is picked up, thereby, it is facilitated for the reinforced applicator 1 to be accurately provided on the periphery of the applicator 2 in sleeving manner and for the reinforced applicator 1 to be smoothly stuck on the skin surface of a body.

In some examples, the first supporting member 121 may be provided along the inner periphery of the adhesion portion 11 (see Fig. 5). In this case, the first supporting member 121 with a relatively large Young's modulus is provided on the inner periphery of the adhesion portion 11, in a result that the deformation resistance of the inner periphery of the reinforced applicator 1 is greater than the deformation resistance of the adhesion portion 11, when the reinforced applicator 1 is used, it is facilitated for the reinforced applicator 1 to be accurately provided on the periphery of the applicator 2 in sleeving manner.

In some examples, the first supporting member 121 may be of annular shape (see Fig.3). In some examples, the second supporting member 124 may also be of annular shape (see Fig.3). Thereby, the deformation resistance of the inner periphery of the reinforced applicator 1 can be further improved, when the reinforced applicator 1 is used, it is facilitated for the reinforced applicator 1 to be accurately provided on the periphery of the applicator 2 in sleeving manner can be facilitated.

In some examples, the inner diameter of the second supporting member 124 may be gradually lessened as away from the upper surface (see Fig.4, Fig.5). That is, the second supporting member 124 may gradually shrink inward along the direction away from the upper surface. In some examples, the inner contour of one end of the second supporting member 124 relatively away from the upper surface may be smaller than the outer contour of the application housing 21. In this case, when the reinforced applicator1 is provided on the periphery of the application housing 21 in sleeving manner and the applicator 2 is stuck on the body surface of a host, the end of the second supporting member 124 relatively away from the upper surface can be stuck and fit to the outer contour of the application housing 21 and applies a force to the applicator 2 towards the body surface of the host, that is, the applicator 2 can be pressed by the second supporting member 124, an accommodating space can be provided for the applicator 2 by the second supporting member 124, the stability of the applicator 2 can be improved when being stuck, thereby displacement or dropping of the applicator 2 in use can be effectively prevented.

In some examples, when the reinforced applicator 1 is provided on the periphery of the application housing 21 in sleeving manner and the applicator 2 is stuck on the body surface of a host, projection is made along the direction parallel to the surface of the adhesion portion 11, the upper surface of the second supporting member 124 may be substantially flush with the upper surface of the application housing 21. In some examples, the height of the second supporting member 124 may be equal to or slightly greater than the thickness of the application housing 21. In some examples, the height of the second bearing member 124 plus the thickness of the adhesion portion 11 plus the thickness of the first supporting member 121 may be equal to or slightly greater than the thickness of the application housing 21. Thereby, the stability of the applicator 2 can be further improved when being stuck.

In some examples, the end of the second supporting member 124 away from the upper surface may be of arc shape (see Fig.4, Fig.5). In this case, the end protruding from the upper surface of the second bearing member 124 away from the upper surface is set to be arc-shaped, enabling the connection of a side of the outer contour of the second supporting member 124 and the upper surface to be transited smoothly, in use of user, the scratches on the reinforced applicator caused by articles, such as clothing etc., can be reduced, thereby the adhesion stability of the reinforced applicator 1 can be improved.

In some examples, the first supporting member 121 may include an upper layer sheet 122 and a lower layer sheet 123 (see Fig.4). In some examples, the upper layer sheet 122 and the lower layer sheet 123 may be of sheet shape. In some examples, the adhesion portion 11 may be clamped by the upper layer sheet 122 and the lower layer sheet 123 (see Fig.4). That is, the adhesion portion 11 may be located between the upper layer sheet 122 and the lower layer sheet 123. In this case, the adhesion portion 11 is clamped by the upper layer sheet 122 and the lower layer sheet 123, the first supporting member 121 and the adhesion portion 11 can be more securely connected. However, the examples of the present disclosure are not limited hereof, in other examples, the upper layer sheet 122 and the lower layer sheet 123 may also be provided on the same surface of the attachment part 11.

In some examples, a contour of the upper layer sheet 122 and a contour the lower layer sheet 123 may be completely consistent. In some examples, the contour of the upper layer sheet 122 and the contour the lower layer sheet 123 may be inconsistent. For example, the contour of the upper layer sheet 122 may be smaller than the contour of the lower layer sheet 123. In some examples, the inner periphery of the adhesion portion 11 may be clamped by the upper layer sheet 122 and the lower layer sheet 123. In some examples, a projection is made along the direction orthogonal to the surface of the adhesion portion 11, the overlapping portion of the upper layer sheet 122 and the lower layer sheet 123 may be partially overlapped on the surface of the adhesion portion 11. In some examples, a projection is made along the direction orthogonal to the surface of the adhesion portion11, the overlapping portion of the upper layer sheet 122 and the lower layer sheet 123 may be completely located within the surface of the adhesion portion 11. Thereby, the adhesion portion can be clamped by the upper layer sheet 122 and the lower layer sheet 123.

In some examples, the second supporting member 124 may include a hollowout 125 formed on the side wall (see Fig.4, Fig.5). In some examples, the hollowout 125 may be of square, circle, and other regular or irregular shape. In some examples, the number of the hollowout 125 may be one or plural. For example, the number of the hollow125 may be 1, 3, 6, or 8. In this case, the internal space of the reinforced applicator 1 can be connected with outside through the hollowout 125, resulting in enabling the fluids to be not easy left at the location where the applicator 2 is stuck on the skin surface of a user, thereby it is facilitated to maintain dry and comfortable status on the applicator 2 area, and the using comfort of the user can be improved.

Fig.6 is a schematic explosive diagram showing the reinforced applicator 1 according to an example of the present disclosure.

In some examples, the reinforced applicator 1 may further include a peeling layer 14 (see Fig. 5, Fig. 6). In some examples, the peeling layer 14 may be provided on another surface opposite to the surface of the adhesive layer 13 having an adhesion portion 11 (see Fig.5). In some examples, at least a portion of the adhesive layer 13 may be covered by the peeling layer. That is, a portion of the adhesive layer 13 may be covered by the peeling layer 14, or the adhesive layer 13 may be completely covered. In this case, the adhesive layer 13 is protected by the peeling layer 14, capable of effectively preventing the adhesive layer 13 from being contaminated before use which may result in the decrease of the adhesion strength of the adhesive layer 13, thereby it is facilitated to maintain the adhesion strength of the adhesive layer 13, resulting in the improvement of the stability when the reinforced applicator 1 is used and stuck on the skin surface of a user.

In some examples, the peeling layer 14 may be an entirety, or may consist of a plurality of portions. For example, the peeling layer 14 may consist of two portions (see Fig.5, Fig.6). In this case, a portion of the peeling layer 14 may be removed first, then a portion of the adhesive layer 13 is stuck on the skin prior to the reinforced applicator 1 being provided on the periphery of the applicator 2 in sleeving manner, and then, the remaining peeling layer 14 may be removed , and the adhesive layer 13 may be stuck on the skin surface completely, the reinforced applicator 1 can be more easily stuck on the skin smoothly when used by the user.

In some examples, the peeling layer 14 may have a peeling wing 141 (see Fig.5, Fig.6). In some examples, the peeling wing 141 may be in the shape of folded outward. In this case, the user may hold the peeling wing 141 and pull towards outside (the direction of D1, D2 as shown in Fig.5), it is facilitated for the peeling layer 14 to be peeled off from the adhesive layer 13, thereby it is facilitated to stick the reinforcement adhesive 1 on the skin surface.

In some examples, the peeling wing 141 may have a holding portion 142 (see Fig.5, Fig.6). In some examples, the holding portion 142 may protrude from the outer periphery of the peeling wing 141 and extend towards outside. In this case, the user may hold the holding part 142, and it is further facilitated to remove the peeling layer 14 from the adhesive layer 13.

In some examples, the inner contour of the peeling layer 14 may be slightly smaller than the inner contour of the adhesion portion 11. In some examples, the inner contour of the peeling layer 14 may be the same as the inner contour of the adhesion portion 11 (see Fig.6). In some examples, the peeling layer 14 may be aligned to the inner contour of the adhesion portion 11. In this case, the adhesion portion 11 is completely covered by the peeling layer 14, it is facilitated to maintain the adhesion strength of the adhesive layer 13, thereby the stability of the reinforced applicator 1 can be improved when being stuck on the skin surface of a user. However, the examples of the present disclosure are not limited hereof, in other examples, the inner contour of the peeling layer 14 may also be slightly larger than the inner contour of the adhesion portion 11.

In some examples, the supporting portion 12, the adhesion portion 11, the adhesive layer 13 and the peeling layer 14 may be stacked up (see Fig.6). In this case, when the reinforced applicator 1 is picked up, the reinforced applicator 1 can be picked up by holding the supporting portion 12 protruding from the surface of the adhesion portion 11, thereby, the convenience of operations can be improved, and meanwhile the undesired deformation of reinforced applicator 1 can be reduced when picking up, thereby it is facilitated for the reinforced applicator 1 to be smoothly stuck on the skin surface, in addition, a certain degree of deformation resistance can be provided for reinforced sticker 1 via the supporting portion 12, thereby the reinforced applicator 1 can be smoothly stuck on the skin surface for a long time, thereby, it is facilitated for the reinforced applicator 1 to be provided on the periphery of the applicator 2 in sleeving manner, and the stability of the applicator 2 stuck on the body surface of a user can be improved.

In summary, the present disclosure is able to provide a reinforced applicator 1 which may facilitate the picking up and accurately reinforcement of an applicator 2 in case of one-handed operation.

Although the present disclosure is described in detail above with reference to the accompanying drawings and examples, it is understood that the above description does not limit the present disclosure in any form. A person skilled in the art can make modifications and variations to the present disclosure as required without deviating from the essence and scope of the present disclosure, and all such modifications and variations fall within the scope of the present disclosure.

## Claims

1. A reinforced applicator, **characterized by** including an adhesion portion, a supporting portion, and an adhesive layer, the adhesion portion has an upper surface and a lower surface which are opposite to each other, the Young's modulus of the supporting portion is greater than the Young's modulus of the adhesion portion and supports the adhesion portion, the supporting portion has a first supporting member provided on the adhesion portion, and a second supporting member formed by extending from the first supporting member along the direction away from the upper surface, the adhesive layer is provided on the lower surface.

2. The reinforced applicator according to claim 1, **characterized in that** the adhesion portion is of embracing structure.

3. The reinforced applicator according to claim 2, **characterized in that** the first supporting member is provided along the inner periphery of the adhesion portion.

4. The reinforced applicator according to claim 2, **characterized in that** the first supporting member and/or the second supporting member are/is of annular shape.

5. The reinforced applicator according to claim 4, **characterized in that** the inner diameter of the second supporting member gradually is tapered out as it is away from the upper surface.

6. The reinforced applicator according to claim 5, **characterized in that** one end of the second supporting member away from the upper surface is of arc-shaped.

7. The reinforced applicator according to claim 1 or 4, **characterized in that** the second supporting member includes a hollowout formed on the side wall.

8. The reinforced applicator according to claim 1, **characterized in that** the first supporting member includes an upper layer sheet and a lower layer sheet which are of sheet shape, the adhesion portion is clamped by the upper layer sheet and the lower layer sheet.

9. The reinforced applicator according to claim 1, **characterized in that** a waterproof layer is provided on the upper surface.

10. The reinforced applicator according to claim 1, **characterized by** further including a peeling layer provided on the adhesive layer, the peeling layer is provided on the surface of the adhesive layer and covers at least a portion of the adhesive layer.
